# FASCICULE DE BREVET EUROPEEN

(11) **EP 0 874 626 B1**
(45) Date de publication et mention de la délivrance du brevet: **29.12.2004**
(21) Numéro de dépôt: 97919115.2
(22) Date de dépôt: 17.09.1997
(51) Int. Cl.: A61K 31/19, A61K 31/415, A61K 31/55, A61K 31/235, A61K 31/38, A61K 31/44, A61K 7/48, A61P 17/02

(54) **UTILISATION DES INHIBITEURS DE L'ACTIVITE DE L'ACIDE RETINOIQUE POUR FAVORISER LA CICATRISATION**
VERWENDUNG VON INHIBITOREN DER RETINSÄUREWIRKUNG ZUR WUNDHEILUNG
USE OF INHIBITORS OF RETINOIC ACID ACTIVITY FOR WOUND HEALING

(30) Priorité: 20.09.1996 FR 9611511
(43) Date de publication de la demande: 04.11.1998
(73) Titulaire: CENTRE INTERNATIONAL DE RECHERCHES DERMATOLOGIQUES GALDERMA (C.I.R.D. GALDERMA), 06560 Valbonne (FR)
(72) Inventeur: DEMARCHEZ, Michel, F-06620 Le Bar sur Loup (FR); ROSSIO, Patricia, F-06130 Plas Cassier (FR); JOMARD, André, F-06460 Saint Vallier de Thiey (FR)
(74) Mandataire: Tezier Herman, Béatrice
(86) Numéro de dépôt international: PCT/FR1997/001641
(87) Numéro de publication internationale: WO 1998/011886

(56) Documents cités:
- EP-A- 0 448 213
- EP-A- 0 465 343
- EP-A- 0 568 898
- EP-A- 0 658 553
- EP-A- 0 661 259
- EP-A- 0 749 755
- WO-A-94/14777
- WO-A-96/30009
- EYROLLES ET AL.: "Retinoid antagonists : molecular design based on the ligand superfamily concept" MED. CELL. RES., vol. 2, 1992, pages 361-367, XP000674651 cité dans la demande
- KANEKO ET AL.: "Retinoid antagonists" MED. CHEM. RES., vol. 1, 1991, pages 220-225, XP000674650 cité dans la demande
- MC DONALD ET AL.: "Effect of retinoic acid on wound healing of laser burns to porcine retinal pigment epithelium" CAN. J. OPHTHALMOL., vol. 31, no. 4, juin 1996, pages 175-178, XP000674653
- KLEIN: "Identification and functional separation of retinoic acid receptor neutral antagonists and inverse agonists" J. BIOL. CHEM., vol. 271, no. 37, 13 septembre 1996, pages 22692-22696, XP002048821
- STANDEVEN: "Specific antagonists of retinoid toxicity in mice" TOXICOL. APPL. PHARMACOL., vol. 138, no. 1, mai 1906, pages 169-173, XP002048822

## Description

La présente invention concerne l'utilisation d'inhibiteurs de l'activité de l'acide rétinoïque, pour la préparation d'une composition phamaceutique pour favoriser la cicatrisation, notamment de la peau.

On sait, d'une manière générale, que l'acide rétinoïque tout-trans (all-trans retinoic acid) agit sur la différenciation et/ou la prolifération des cellules en interagissant avec des récepteurs nucléaires ou RARs (Retinoic Acid Receptors) contenus dans le noyau cellulaire. De nombreux analogues structuraux synthétiques de l'acide rétinoïque tout-trans ou de l'acide 9-cis-rétinoïque, couramment dénommés "rétinoïdes", ont été décrits à ce jour dans la littérature. Il existe, à ce jour, trois sous-types identifiés de récepteurs RAR appelés respectivement RAR-α, RAR-β et RAR-γ. Ces récepteurs, après fixation du ligand (i.e. de l'acide rétinoïque tout-trans), interagissent avec la région promotrice de gènes régulés par l'acide rétinoïque au niveau d'éléments de réponses spécifiques (RARE).

Certains analogues peuvent se fixer et activer un sous-type de récepteur RAR (α, β ou γ) particulier. D'autres analogues, enfin, ne présentent aucune activité sélective particulière vis-à-vis de ces différents récepteurs. A cet égard, et par exemple, l'acide rétinoïque tout-trans active les RARs (ligand agoniste spécifique RARs), tous sous-types confondus.

L'acide rétinoïque et les rétinoïdes en général, ont été revendiqués pour traiter les désordres ou affections suivants: les acnés vulgaires, comédoniennes, polymorphes, rosacées, les acnés nodulokystiques, conglobata, les acnés séniles, les acnés secondaires telles que l'acné solaire, médicamenteuse ou professionnelle; les autres types de troubles de la kératinisation, notamment les ichtyoses, les états ichtyosiformes, la maladie de Darrier, les kératodermies palmoplantaires, les leucoplasies et les états leucoplasiformes, le lichen cutané ou muqueux (buccal); les autres affections dermatologiques liées à un trouble de la kératinisation avec une composante inflammatoire et/ou immuno-allergique et notamment toutes les formes de psoriasis qu'il soit cutané, muqueux ou unguéal, et même le rhumatisme psoriatique, ou encore l'atopie cutanée, telle que l'eczéma ou l'atopie respiratoire ou encore l'hypertrophie gingivale; certaines affections inflammatoires ne présentant pas de trouble de la kératinisation, telles que l'arthrite; les proliférations dermiques ou épidermiques qu'elles soient bénignes ou malignes, qu'elles soient ou non d'origine virale telles que verrues vulgaires, les verrues planes et l'épidermodysplasie verruciforme, les papillomatoses orales ou florides et les proliférations pouvant être induites par les ultra-violets notamment dans le cas des épithélioma baso et spinocellulaires; les autres désordres dermatologiques tels que les dermatoses bulleuses et les maladies du collagène; certains troubles ophtalmologiques, notamment les cornéopathies; le vieillissement de la peau, qu'il soit photo-induit ou chronologique ou les pigmentations et les kératoses actiniques, ou toutes pathologies associées au vieillissement chronologique ou actinique; les stigmates de l'atrophie épidermique et/ou dermique induite par les corticostéroïdes locaux ou systémiques, ou toute autre forme d'atrophie cutanée; les troubles de la cicatrisation ou les vergetures; les troubles de la fonction sébacée tels que l'hyperséborrhée de l'acné ou la séborrhée simple; les états cancéreux ou précancéreux; les affection d'origine virale au niveau cutané ou général (virus d'immunodéficience humain : HIV-1 ou virus de l'hépatite B; l'alopécie; les affections du système cardiovasculaire telles que l'artériosclérose.

L'acide rétinoïque et les rétinoïdes en général, en se liant avec les récepteurs RARs permettent de réguler l'activité des récepteurs RARs et de traiter les désordres ou affections ci-dessus.

Les antagonistes de l'acide rétinoïque vont au contraire inhiber l'activité de l'acide rétinoïque ou ses métabolites au niveau cellulaire. Il s'agit plus particulièrement des antagonistes RARs qui viennent se lier aux récepteurs RARs, mais n'induisent pas l'activité observée pour l'acide rétinoïque ou les rétinoïdes.

Ainsi, il a été montré que des antagonistes des récepteurs RARα inhibent la différenciation cellulaire induite par les rétinoïdes sur les cellules de la lignée cellulaire HL60 ou au contraire réversent l'inhibition de la prolifération des cellules-B de la souris induite par les rétinoïdes (C. APFEL & al., Proc. Natl. Acad. Sci. USA, 89, 1992, 7129-7133).

Différents antagonistes de l'acide rétinoïque sont décrits dans la demande de brevet EP 568 898. Ils sont préconisés pour traiter les problèmes de surrégulation des récepteurs RARs, en particulier les affections immunologiques, auto-immunes ou les affections présentant une forte composante immunologique comme le psoriasis.

L'influence néfaste d'une carence en vitamine A sur la cicatrisation de la peau est décrite par REED & CLARK en 1985 (B.R. REED & R.A.F. CLARK, J. Am. Acad. Dermatol., 13, 6, 1985,919-941).

La nécessité de l'acide rétinoïque dans le développement normal de la peau a été confirmée par l'expression d'un récepteur dominant négatif de l'acide rétinoïque chez la souris (M. SAITOU, Nature, 374, 1995, 159). Il a été montré que la carence d'activité de l'acide rétinoïque entraînait une inhibition du développement de la peau avec une suppression de la maturation de l'épiderme.

Compte-tenu de cette nécessité et des connaissances sur l'activité des rétinoïdes et des antagonistes on pouvait donc supposer que ces antagonistes de l'acide rétinoïque en inhibant son activité au niveau cellulaire pouvaient traiter les troubles de la cicatrisation en ralentissant cette dernière, alors que les rétinoïdes l'accéléreraient.

En effet, la cicatrisation, plus particulièrement la cicatrisation cutanée est un processus complexe ou les différents événements dermiques et/ou épidermiques sont étroitement imbriqués, et comprennent les différentes phases suivantes: formation d'une croûte, production d'un tissu de granulation et rapprochement des berges de la plaie, et formation d'un épiderme puis d'un néoderme.

Lorsqu'une plaie dermo-épidermique est induite, il y a une première phase inflammatoire qui a pour but majeur de nettoyer la plaie avec formation d'une croûte qui l'isole de l'environnement extérieur.

Sous cette croûte va ensuite se développer un tissu de granulation essentiellement formé de vaisseaux sanguins et de fibroblastes spécialisés appelés "myofibroblastes". Ce sont des cellules qui vont participer au phénomène de rétraction de la plaie qui a pour but de rapprocher les berges de la plaie.

Entre la croûte et le tissu de granulation, sur ce dernier, les kératinocytes issus des berges de la plaie vont migrer pour reformer un épiderme: c'est le phénomène de réépithélialisation.

Sous l'épiderme reconstruit, le tissu de granulation est passé d'un état cellulaire (riche en cellules) à un état fibreux (riche en matrice extracellulaire). Par la suite, ce tissu fibreux cicatriciel est envahi par des fibroblastes issus du derme périphérique non lésé qui vont reconstruire le néoderme.

La rétraction de la plaie n'est pas une étape nécessaire à la cicatrisation. Elle a pour but essentiel de diminuer l'espace à "reépidermiser" entre les berges de la plaie. Dans certains cas, cette rétraction de la plaie peut être la cause de troubles importants en induisant des pertes de fonction, notamment à proximité des orifices ou des articulations, par exemple dans le cas des brûlures des mains.

Il est donc important de pouvoir disposer de nouveaux composés qui permettent de favoriser la cicatrisation, en particulier la cicatrisation cutanée, c'est à dire qui favorisent la reépidermisation (reépithélialisation et normalisation de l'épiderme et du derme) et/ou qui limitent le phénomène de rétraction de la plaie.

Or, contrairement à ce que l'on pouvait attendre compte-tenu des connaissances sur la nécessité de l'acide rétinoïque pour la maturation de l'épiderme et la croissance de la peau, nous avons maintenant pu mettre en évidence le fait que l'inhibition de l'activité de l'acide rétinoïque ou de ses métabolites, et plus particulièrement de l'activité de l'acide rétinoïque endogène, permet de favoriser la cicatrisation, alors que les rétinoïdes en renforçant l'activité de l'acide rétinoïque endogène ralentissent le comblement de la plaie. Cette constatation est d'autant plus remarquable qu'elle a été faite sur des peaux normales, c'est à dire qui ne présentent pas de troubles de la cicatrisation qui auraient été liés à une hypervitaminose A.

La présente invention concerne donc l'utilisation d'au moins un inhibiteur de l'activité de l'acide rétinoïque pour la préparation d'une composition pharmaceutique, l'inhibiteur de l'activité de l'acide rétinoïque ou la composition pharmaceutique étant destiné à favoriser la cicatrisation, en particulier la cicatrisation cutanée.

Les inhibiteurs de l'activité de l'acide rétinoïque peuvent agir selon deux voies, la première en accélérant le métabolisme cellulaire de l'acide rétinoïque de manière à diminuer la concentration cellulaire de ce dernier, la seconde en antagonisant son action au niveau cellulaire.

Les inhibiteurs de l'activité de l'acide rétinoïque peuvent donc être selon l'invention des accélérateurs du métabolisme de l'acide rétinoïque ou des antagonistes, agonistes inverses ou agonistes partiels de l'acide rétinoïque.

Par antagonistes de l'acide rétinoïque on entend de préférence selon l'invention les composés qui inhibent l'action de l'acide rétinoïque et/ou de ses métabolites et/ou des rétinoïdes. Il s'agit plus particulièrement des antagonistes RARs, qui se fixent aux récepteurs RARs sans toutefois les activer.

On entend plus particulier les composés qui inhibent l'activité de l'acide rétinoïque "in vivo" sélectionnés selon le test décrit dans la demande de brevet EP 96 401 170 déposée le 31 mai 1996 au nom du CIRD GALDERMA. Il s'agit d'un procédé pour identifier des molécules antagonistes des RARs, caractérisé en ce qu'il comprend les étapes suivantes : (i) on applique topiquement sur une partie de la peau d'un mammifère une quantité suffisante d'une molécule agoniste des RARs, (ii) on administre par voie systémique ou topique sur ce même mammifère ou sur cette même partie de la peau du mammifère, avant, pendant ou après l'étape (i), une molécule susceptible de présenter une activité antagoniste des RARs et (iii) on évalue la réponse sur la partie de la peau ainsi traitée du mammifère. Ainsi, lorsque la molécule administrée est un antagoniste des RARs, l'augmentation de l'épaisseur de la partie de la peau traitée du mammifère par une molécule agoniste des RARs n'est pas constatée ou est diminuée. On observe donc une inhibition de la réponse. De manière pratique, le mammifère est un rongeur tel qu'une souris, un rat, un cobaye, un hamster ou un lapin. La partie de la peau de mammifère utilisée peut être n'importe quelle partie du corps du mammifère. La réponse sur la partie de la peau ainsi traitée du mammifère à évaluer correspond à une modification clinique de celle-ci. De manière générale, cette réponse à évaluer correspond à une modification de l'épaisseur de la partie de la peau ainsi traitée. La mesure de l'épaisseur de la partie de la peau ainsi traitée peut être réalisée par toute méthode connue en soi. Lorsque la partie de la peau utilisée est lisse, on peut mesurer son épaisseur en la pliant. De manière plus pratique, on utilise la peau de l'oreille. La mesure de l'épaisseur de l'oreille peut alors être réalisée par un micromètre "oditest". Bien entendu, l'évaluation de l'étape (iii) correspond à une mesure de la réponse de la partie de la peau ainsi traitée et à une comparaison de cette mesure avec celle de la réponse de cette même partie de la peau traitée, dans les mêmes conditions, avec la molécule agoniste des RARs seule. De préférence, les molécules agonistes des RARs sont choisis parmi les composés capables d'induire la différenciation des cellules (F9) du tératocarcinome embryonnaire de souris. La sécrétion de l'activateur plasminogène qui accompagne cette différenciation est un indice de la réponse biologique des cellules F9 à ces composés. On sait également que la capacité de ces composés à induire l'activateur plasrminogène est corrélée directement avec l'affinité et l'activité qu'ils ont sur les récepteurs RARs endogènes aux cellules F9 (Skin Pharmacol., 1990, 3, pp. 256-267). Parmi les molécules agonistes des RARs induisant la différenciation des cellules F9, on peut plus particulièrement citer :
- l'acide rétinoïque *tout-trans*,
- l'acide 2-(5,6,7,8-tetrahydro -5,5,8,8-tetraméthyl -2-naphtyl) -6-benzo[b]thiophen carboxylique,
- l'acide 4-[(5,6,7,8-tétrahydro -5,5,8,8-tétraméthyl -2-naphtyl) carboxamido] benzoïque,
- l'acide 4-[(5,6,7,8-tétrahydro -5,5,8,8-tétraméthyl -2-naphtyl) carbamoyl] benzoïque.
Dans ce qui suit ou ce qui précède, on entend par voie topique, toute technique d'administration d'un produit par application directe de ce dernier sur une partie superficielle (ou externe) du corps, et par voie systémique, toute technique d'administration d'un produit par une voie autre que topique, par exemple entérale et/ou parentérale. Dans le cas de la voie systémique, on préfère utiliser la voie orale. La quantité suffisante d'une molécule agoniste des RARs à appliquer correspond à celle à laquelle on observe une réponse de la partie traitée de la peau du mammifère après l'étape (i). Ainsi, de préférence et selon la nature de la molécule agoniste des RARs utilisée, cette quantité varie entre 0,0001 % et 2% en poids par volume de solution appliquée.

Les antagonistes de l'acide rétinoïque sont notamment des composés décrits dans les demandes de brevet EP 661 259, EP 740 937, EP 658 553, EP 568 898, WO 95/33745, WO 97/09297 et WO 94/14777, et dans plusieurs publications scientifiques, notamment EYROLLES & al (J. Med. Chem., 37, 1994, 1508-1517; Med. Chem. Res., 2, 1992, 361-367) et KANEKO & al (Med. Chem. Res., 1, 1991, 220-225) et qui inhibent l'action de l'acide rétinoïque et/ou des rétinoïdes

Les antagonistes RARs utiles selon l'invention sont en particulier sélectionnés parmi les composés suivants:
- acide 4-[7-(1-adamantyl) -6-méthoxyéthoxyméthoxy -2-naphtyl] benzoïque,
- acide 4-{[5,6-dihydro -5,5-diméthyl -8-(4-méthylphényl) -2-naphtalenyl] éthynyl} benzoïque,
- acide (E)-4-[2-(4,4-diméthyl -7-heptyloxy -1,1-dioxo -3,4-dihydro -2H-1-benzothiopyran -6-yl) propenyl] benzoïque,
- acide 4-[4,5,7,8,9,10-hexahydro -7,7,10,10-tétraméthyl -1-(3-pyridylméthyl) anthra[1,2-b]pyrrol -3-yl] benzoïque,
- acide 4-[4,5,7,8,9,10-hexahydro -7,7,10,10-tétraméthyl -1-(3-pyridylméthyl) thioanthra[1,2-b]pyrrol -3-yl] benzoïque,
- acide 4-[4,5,7,8,9,10-hexahydro -7,7,10,10-tétraméthyl -1-(3-pyridylméthyl) anthra[1,2-d]pyrazol -3-yl] benzoïque,
- acide 4-[3-(diamantyl) -4-méthoxybenzamido] benzoïque,
- acide 4-[3-(diamantyl) -4-méthoxybenzoyloxy] benzoïque,
- acide 4-(N-phényl -5,6,7,8-tétrahydro -5,5,8,8-tétraméthylnaphto[2,3-d]imidazal-2-yl)benzoïque,
- acide 4-(N-benzyl -5,6,7,8-tétrahydro -5,5,8,8-tétraméthylnaphto[2,3-d]imidazol-2-yl)benzoïque,
- acide 4-(5H-7,8,9,10-tetrahydro -5,7,7,10,10-pentaméthylbenzo[c] -naphto[2,3-b] [1,4]diazepin -3-yl)benzoïque,
- acide 4-[1-(1-méthoxy-2,2,2-trifluoroéthyl) -5,6,7,8-tétrahydro -5,5,8,8-tétraméthyl -3-anthracényl]benzoïque,
- acide 4-(5,5-diméthyl-8-phényl-5,6-dihydro-naphtalen-2-yléthynyl)benzoïque,
- acide 4-(5,5-diméthyl-8-p-tolyl-5,6-dihydro-naphtalen-2-yléthynyl)benzoïque.
   Dans le cadre de l'invention, les inhibiteurs de l'activité de l'acide rétinoïque peuvent être avantageusement employés en combinaison avec d'autres composés actifs, en particulier utiles pour favoriser la cicatrisation, qui ne viennent pas contrarier l'activité de l'inhibiteur de l'activité de l'acide rétinoïque pour favoriser la cicatrisation. Il s'agit notamment de ,composés tels que la vitamine C, la vitamine K, la vitamine B, la vitamine E ou leurs analogues; les agents désinfectants; les antibiotiques administrés par voie topique ou systémique comme l'érythromycine et ses esters, la néomycine, la gentamycine, la clindamycine et ses esters, les tétracyclines ; les agents antifongiques tels que le kétoconazole ou les polyméthylène-4,5 isothiazolidones-3; les agents antimicrobiens topiques, notamment les iodophores, les complexes ou les sels d'argent, de cuivre ou de zinc, le peroxyde de benzoyle, les extraits d'Aloe vera, les facteurs de croissances; la sphingosylphosphorylcholine, etc.
   Dans ce cas, l'inhibiteur de l'activité de l'acide rétinoïque et les autres composés actifs peuvent être administrés ensemble dans une même composition cosmétique ou pharmaceutique, ou encore séparément dans des compositions séparées, de manière simultanée ou décalée dans le temps.
   La composition pharmaceutique comprenant au moins un inhibiteur de l'activité de l'acide rétinoïque comprend un support cosmétiquement ou pharmaceutiquement acceptable et compatible avec le mode d'administration retenu.
   La quantité d'inhibiteur de l'activité de l'acide rétinoïque est une quantité efficace dépendant bien entendu du traitement désiré et de la nature du composé choisi; elle est donc déterminée par l'homme du métier.
   L'administration des composés selon l'invention peut être effectuée par voie systémique (notamment entérale ou parentérale), topique ou oculaire.
   Par voie entérale, la composition est une composition pharmaceutique, pouvant se présenter sous forme de comprimés, de gélules, de dragées, de sirops, de suspensions, de solutions, de poudres, de granulés, d'émulsions, de microsphères ou de nanosphères ou de vésicules lipidiques ou polymériques permettant une libération contrôlée. Par voie parentérale, la composition, plus particulièrement la composition pharmaceutique, peut se présenter sous forme de solutions, d'émulsions ou de suspensions pour perfusion ou pour injection.
   Les inhibiteurs de l'activité de l'acide rétinoïque, plus particulièrement les antagonistes de l'acide rétinoïque sont généralement administrés à une dose journalière d'environ 0,01 mg/kg à 100 mg/kg en poids corporel, et ceci à raison de 1 à 3 prises.
   Par voie topique, la composition est une composition pharmaceutique, plus particulièrement destinée au traitement de la peau et/ou des muqueuses. Elle peut alors se présenter sous forme de solutions ou de suspensions, d'onguents, de pommades, de crêmes, de laits, de gels, de poudres, de tampons imbibés, de lotions, de sprays, ou de produits moussants
ou nettoyants. Elle peut également se présenter sous forme de microsphères ou nanosphères ou vésicules lipidiques ou polymériques ou de patches polymériques, d'hydrogels ou de pansements permettant une libération contrôlée. Cette composition par voie topique peut par ailleurs se présenter soit sous forme anhydre, soit sous une forme aqueuse.

La composition topique peut également comprendre des excipients appropriés pour former un film à la surface de la peau, soit pour isoler la plaie à cicatriser de l'environnement extérieur et éviter les contaminations, soit pour former une matrice hydrophobe qui attire par osmose les fluides, les bactéries et les débris de tissus.

Par voie oculaire, la composition peut alors se présenter sous forme d'onguents, de crêmes, de gels ou de collyres appropriés pour cette application spécifique.

Cette composition à usage topique ou oculaire contient au moins un antagoniste de l'acide rétinoïque à une concentration de préférence comprise entre 0,001% et 5% en poids par rapport au poids total de la composition.

La composition selon l'invention peut en outre contenir des additifs inertes ou même pharmacodynamiquement actifs ou des combinaisons de ces additifs, et notamment: des agents mouillants; des émollients; des agents hydratants comme le glycérol, le PEG 400, la thiamorpholinone, et ses dérivés ou bien encore l'urée; des agents anti-inflammatoires non stéroïdiens ; des caroténoïdes et, notamment, le β-carotène.

La composition peut également contenir des agents d'amélioration de la saveur, des agents conservateurs tels que les esters de l'acide parahydroxybenzoïque, les agents stabilisants, des agents régulateurs d'humidité, des agents régulateurs de pH, des agents modificateurs de pression osmotique, des agents émulsionnants, des filtres UV-A et UV-B, des antioxydants hydrophiles ou lipophiles, tels que l'α-tocophérol, le butylhydroxyanisole ou le butylhydroxytoluène, des chélatants comme l'EDTA et ses sels.

Les compositions selon l'invention sont particulièrement utiles pour améliorer la cicatrisation, en particulier pour le traitement des plaies chirurgicales, notamment les plaies induites par la chirurgie plastique et/ou par les peelings obtenus par voie chimique (acide), par dermoabrasion ou par laser, et/ou les plaies traumatiques, notamment les coupures ou brulures de toutes sortes, telles que les brulures induites par les U.V.

Les exemples ci-après permettent d'illustrer l'invention sans toutefois chercher à en limiter la portée.

### Exemple 1 Etude de l'activité des antagonistes de l'acide rétinoïque sur la cicatrisation

Cet exemple a pour but de mettre en évidence l'activité d'un antagoniste RAR dans le processus de cicatrisation d'une plaie cutanée induite expérimentalement.

### Méthode

Les études sont effectuées sur la peau humaine transplantée chez la souris nude selon la méthode décrite par DEMARCHEZ & al (M. DEMARCHEZ & al, Develop. Biology, 113, 1986, 90-96; M. DEMARCHEZ & al, Develop. Biology, 121, 1987, 119-129).

Une plaie profonde est réalisée sur la peau humaine transplantée chez la souris nude, en pratiquant l'exérèse de la peau au centre du greffon à l'aide d'un punch de 2 mm. La plaie est ensuite laissée à l'air libre.

L'acide 4-[7-(1-adamantyl) -6-méthoxyéthoxyméthoxy -2-naphtyl] benzoïque (antagoniste RAR) en suspension dans le crémophore EL 25% à la dose de 30 mg/kg (produit testé), ou le véhicule seul (témoin), est administré per os par gavage sous un volume de 10 mg/kg.

Le traitement est administré une fois par jour pendant 7 jours à partir du jour d'induction de la plaie cutanée (traitement immédiat) chez 8 souris (4 dans le groupe du produit testé et 4 dans le groupe témoin) et une fois par jour pendant 7 jours en débutant 7 jours après induction de la plaie cutanée (traitement différé)chez 10 souris (5 dans le groupe produit testé et 5 dans le groupe témoin).

24 heures après le dernier traitement, les souris sont anesthésiées et la perte insensible en eau (p.i.e.) est mesurée au niveau du greffon puis les souris sont euthanasiées. Les greffons sont coupés à la limite de la zone cicatricielle et congelés dans l'azote liquide.

Les échantillons sont coupés au cryostat à 5µm, de manière à obtenir des coupes sériées sur l'ensemble de la zone de plaie.

Une coloration hémalum-éosine-safran est réalisée pour l'examen histologique de la zone cicatricielle. Des marquages à l'aide d'anticorps selon la méthode d'immunofluorescence sont réalisés pour l'étude du processus de cicatrisation dans le derme et dans l'épiderme à différents niveaux de la zone de plaie. Les anticorps suivants sont utilisés: anti involucrine humaine (différenciation épidermique), AE1 (kératines de la couche basale), anti kératine K10, anti vimentine humaine (cellules d'origine mésenchymateuse du derme et de l'épiderme), TMH1 (mélanocytes), anti HLADR (cellules de Langerhans), anti facteur VIII (vaisseaux sanguins), anti collagene IV (collagène des emmbranes basales).

Les mesures et observations effectuées dans le groupe traité avec l'acide 4-[7-(1-adamantyl) -6-méthoxyéthoxyméthoxy -2-naphtyl] benzoïque sont exprimées par rapport aux mesures et observations effectuées dans le groupe témoin (traité avec le véhicule).

### Résultats

Traitement immédiat: Après 7 administrations par voie orale dans le modèle de cicatrisation de la peau humaine transplantée chez la souris nude, en débutant le traitement le jour de l'induction de la plaie, l'acide 4-[7-(1-adamantyl) -6-méthoxyéthoxyméthoxy -2-naphtyl] benzoïque ne modifie pas de manière statistiquement significative la p.i.e. et n'induit pas de modification morphologique au niveau de la zone non lésée du greffon, mais il normalise la différenciation épidermique et stimule la réapparition des cellules de Langerhans dans les zones de bordure de greffe et de bordure de plaie.

Dans la zone lésée, on observe une progression plus avancée des langues épidermiques, une différenciation améliorée, la présence de mélanocytes et de cellules de Langerhans en périphérie, un tissu conjonctif plus évolué et une diminution de rétraction de la plaie.

Ces données indiquent que l'acide 4-[7-(1-adamantyl) -6-méthoxy éthoxyméthoxy -2-naphtyl] benzoïque favorise la reconstruction des tissus dermiques et épidermiques.
Traitement différé Après 7 administrations par voie orale d'acide 4-[7-(1-adamantyl) -6-méthoxyéthoxyméthoxy -2-naphtyl] benzoïque dans le modèle de cicatrisation de la peau humaine transplantée chez la souris nude, en débutant le traitement 7 jours après l'induction de la plaie, l'étude morphologique montre que la plaie est complètement reépithélialisée et qu'elle n'est pas rétractée. On observe une normalisation de la différenciation de l'épiderme à partir des bords de la plaie et une bonne cohésion de la jonction dermo-épidermique.

Ces résultats montrent clairement que les inhibiteurs de l'activité de l'acide rétinoïque ou de ses métabolites, et plus particulièrement les antagonistes RARs favorisent la reépithélialisation d'une plaie cutanée et par son activité pro-différenciante, accélère la normalisation de l'épiderme reconstruit, tout en limitant le phénomène de rétraction de la plaie.

### Exempte 2 Compositions

Les compositions ci-après sont des exemples de compositions susceptibles d'être employées pour appliquer ou administrer des antagonistes de l'acide rétinoïque dans le but de favoriser la cicatrisation.

### A- VOIE ORALE

(a) Comprimé 0,2 g à libération immédiate (granulation)
   - Antagoniste de l'acide rétinoïque 0,0010 g
   - Lactose codex 0,1204 g
   - Cellulose microcristalline (Avicel PH 101) 0,0640 g
   - Polyvinyl-pyrrolidone (Kollidon K30) 0,0060 g
   - Silice colloïdale (Aerosil 200) 0,0006 g
   - Stéarate de magnésium 0,0020 g
   - Glycolate d'amidon sodique (Explotab) 0,0060 g
   - Eau purifiée qsp granulation
(b) Comprimé 0,8 g à libération immédiate (compression directe)
   - Antagoniste de l'acide rétinoïque 0,0800 g
   - Amidon modifié (Starch 1500) 0,2984 g
   - Cellulose microcristalline (Avicel PH 102) 0,4000 g
   - Silice colloïdale (Aerosil 200) 0,0016 g
   - Sréarate de magnésium 0,0040 g
   - Croscarmellose sodique (Ac-Di-Sol) 0,0160 g
(c) Gelule 0,2 g (capsule molle ou gélule remplie de liquide)
   - Antagoniste de l'acide rétinoïque 0,0050 g
   - Glycérides polyglycosylés insaturés ou huile végétale (soja, maïs, ...) 0,1845 g
   - Cire blanche d'abeille ou huile de ricin hydrogénée 0,0100 g
   - BHT 0,0001 g
   - dl-α-tocophérol 0,0004 g
(d) Solution buvable en ampoules de 5 ml
   - Antagoniste de l'acide rétinoïque 0,0001 g
   - Glycérol 0,7500 g
   - Sorbitol 1,0000 g
   - Propylène glycol 1,0000 g
   - Polyvinyl pyrrolidone (Kollidon K25) 0,5000 g
   - Cyclamate de sodium 0,0050 g
   - Parahydroxybenzoate de sodium 0,0040 g
   - Arome qs
   - Eau purifiée qsp 5 ml

### B- VOIE TOPIQUE

(a) Onguent
   - Antagoniste de l'acide rétinoïque 0,020 g
   - Myristate d'isopropyle 81,700 g
   - Huile de vaseline fluide 9,100 g
   - Silice ("Aérosil 200" vendue par DEGSSA) 9,180 g
(b) Onguent
   - Antagoniste de l'acide rétinoïque 0,300 g
   - Vaseline blanche codex qsp 100 g
(c) Crème Eau-dans-Huile non ionique
   - Antagoniste de l'acide rétinoïque 0,100 g
   - Mélange d'alcools de lanoline émulsifs, de cires et d'huiles ("Eucerine anhydre" vendu par BDF) 39,900 g
   - Parahydroxybenzoate de méthyle 0,075 g
   - Parahydroxybenzoate de propyle 0,075 g
   - Eau déminéralisée stérile qsp 100 g
(d) Lotion
   - Antagoniste de l'acide rétinoïque 0,100 g
   - Polyéthylène glycol (PEG 400) 69,900 g
   - Ethanol à 95% 30,000 g
(e) Crème Huile-dans-Eau non ionique
   - Antagoniste de l'acide rétinoïque 1,000 g
   - Alcool cétylique 4,000 g
   - Monostéarate de glycérole 2,500 g
   - Stéarate de PEG 50 2,500 g
   - Beurre de karité 9,200 g
   - Propylène glycol 2,000 g
   - Parahydroxybenzoate de méthyle 0,075 g
   - Parahydroxybenzoate de propyle 0,075 g
   - Eau déminéralisée stérile qsp 100 g

## Revendications

1. Utilisation d'un inhibiteur de l'activité de l'acide rétinoïque pour la préparation d'une composition pharmaceutique destinée à favoriser la cicatrisation, en particulier la cicatrisation cutanée.

2. Utilisation selon la revendication 1, **caractérisée en ce que** l'inhibiteur de l'activité de l'acide rétinoïque est un antagoniste de l'acide rétinoïque.

3. Utilisation selon la revendication 2, **caractérisée en ce que** les antagonistes de l'acide rétinoïque sont sélectionnés parmi les composés suivants:
- acide 4-[7-(1-adamantyl) -6-méthoxyéthoxyméthoxy -2-naphtyl] benzoïque,
- acide 4-{[5,6-dihydro -5,5-diméthyl -8-(4-méthylphényl) -2-naphtalenyl] éthynyl} benzoïque,
- acide (E)-4-[2-(4,4-diméthyl -7-heptyloxy -1,1-dioxo -3,4-dihydro -2H-1-benzothiopyran -6-yl) propenyl] benzoïque,
- acide 4-[4,5,7,8,9,10-hexahydro -7,7,10,10-tétraméthyl -1-(3-pyridylméthyl) anthra[1,2-b]pyrrol -3-yl] benzoïque,
- acide 4-[4,5,7,8,9,10-hexahydro -7,7,10,10-tétraméthyl -1-(3-pyridylméthyl) thioanthra[1,2-b]pyrrol -3-yl] benzoïque,
- acide 4-[4,5,7,8,9,10-hexahydro -7,7,10,10-tétraméthyl -1-(3-pyridylméthyl) anthra[1,2-d]pyrazol -3-yl] benzoïque,
- acide 4-[3-(diamantyl) -4-méthoxybenzamido] benzoïque,
- acide 4-[3-(diamantyl) -4-méthoxybenzoyloxy] benzoïque,
- acide 4-(N-phényl -5,6,7,8-tétrahydro -5,5,8,8-tétraméthylnaphto[2,3-d]imidazol-2-yl)benzoïque,
- acide 4-(N-benzyl -5,6,7,8-tétrahydro -5,5,8,8-tétraméthylnaphto[2,3-d]imidazol-2-yl)benzoïque,
- acide 4-(5H-7,8,9,10-tétrahydro -5,7,7,10,10-pentaméthylbenzo[c] -naphto[2,3-b] [1,4]diazepin -3-yl)benzoïque;
- acide 4-[1-(1-méthoxy-2,2,2-trifluoroéthyl) -5,6,7,8-tétrahydro -5,5,8,8-tétraméthyl -3-anthracényl]benzoïque,
- acide 4-(5,5-diméthyl-8-phényl-5,6-dihydro-naphtalen-2-yléthynyl)benzoïque.

4. Utilisation selon l'une des revendications 1 à 3, **caractérisée en ce que** la composition pharmaceutique comprend d'autres composés actifs.

5. Utilisation selon l'une des revendications 1 à 4, **caractérisée en ce que** la composition pharmaceutique comprend un support cosmétiquement ou pharmaceutiquement acceptable et compatible avec Je mode d'administration retenu.

6. Utilisation selon l'une des revendications 1 à 5, **caractérisée en ce que** la composition est une composition pharmaceutique, et peut se présenter sous forme de comprimés, de gélules, de dragées, de sirops, de suspensions, de solutions, de poudres, de granulés, d'émulsions, de microsphères ou de nanosphères ou de vésicules lipidiques ou polymériques permettant une libération contrôlée, de solutions ou de suspensions pour perfusion ou pour injection.

7. Utilisation selon la revendication 6, **caractérisée en ce que** les inhibiteurs de l'activité de l'acide rétinoïque sont administrés à une dose journalière d'environ 0,01 mg/kg à 100 mg/kg en poids corporel, et ceci .à raison de 1 à 3 prises.

8. Utilisation selon l'une des revendications 1 à 5, **caractérisée en ce que** la composition pharmaceutique est appropriée pour une administration par voie topique et peut se présenter sous forme de solutions ou de suspensions, d'onguents, de pommades, de crêmes, de laits, de gels, de poudres, de tampons imbibés, de lotions, de sprays, ou de produits moussants ou nettoyants, sous forme de microsphères ou nanosphères ou vésicules lipidiques ou polymériques ou de patches polymériques, d'hydrogels ou de pansements permettant une libération contrôlée, soit sous forme anhydre, sous une forme aqueuse, pouvant également comprendre des excipients appropriés pour former un film à la surface de la peau.

9. Utilisation selon l'une des revendications 1 à 5, **caractérisée en ce que** la composition est une composition appropriée pour une administration par voie oculaire.

10. Utilisation selon l'une des revendications 8 ou 9, **caractérisée en ce que** la quantité d'inhibiteur de l'activité de l'acide rétinoïque est comprise entre 0,001% et 5% en poids par rapport au poids total de la composition.

11. Utilisation selon l'une des revendications 1 à 10, pour le traitement de des plaies chirurgicales ou traumatiques.

## Patentansprüche

1. Verwendung eines Inhibitors der Wirkung von Retinsäure für die Herstellung einer pharmazeutischen Zusammensetzung, die dafür vorgesehen ist, die Wundheilung, insbesondere die Wundheilung der Haut, zu fördern.

2. Verwendung nach Anspruch 1, **dadurch gekennzeichnet, dass** der Inhibitor der Wirkung von Retinsäure ein Antagonist der Retinsäure ist.

3. Verwendung nach Anspruch 2, **dadurch gekennzeichnet, dass** die Antagonisten der Retinsäure unter den folgenden Verbindungen ausgewählt werden:
- 4-[7-(1-Adamantyl)-6-methoxyethoxymethoxy-2-naphthyl]-benzoesäure,
- 4-{[5,6-Dihydro-5,5-dimethyl-8-(4-methylphenyl)-2-naphthalinyl]-ethinyl}-benzoesäure,
- (E)-4-[2-(4,4-Dimethyl-7-heptyloxy-1,1-dioxo-3,4-dihydro-2H-1-benzothiopyran-6-yl)-propenyl]-benzoesäure,
- 4-[4,5,7,8,9,10-Hexahydro-7,7,10,10-tetramethyl-1-(3-pyridylmethyl)-anthra[ 1,2-b]pyrrol-3-yl]-benzoesäure,
- 4-[4,5,7,8,9,10-Hexahydro-7,7,10,10-tetramethyl-1-(3-pyridylmethyl)-thioanthra[1,2-b]pyrrol-3-yl]-benzoesäure,
- 4-[4,5,7,8,9,10-Hexahydro-7,7,10,10-tetramethyl-1-(3-pyridylmethyl)-anthra[ 1,2-d]pyrrol-3-yl]-benzoesäure,
- 4-[3-(Diamantyl)-4-methoxybenzamido]-benzoesäure,
- 4-[3-(Diamantyl)-4-methoxybenzoyloxy]-benzoesäure,
- 4-(N-Phenyl-5,6,7,8-tetrahydro-5,5,8,8-tetramethylnaphtho[2,3-d]imidazol-2-yl)-benzoesäure,
- 4-(N-Benzyl-5,6,7,8-tetrahydro-5,5,8,8-tetramethylnaphtho[2,3-d]imidazol-2-yl)-benzoesäure,
- 4-(5H-7,8,9,10-Tetrahydro-5,7,7,10,10-pentamethylbenzo[c]-naphtho[2,3-b]-[1,4]diazepin-3-yl)-benzoesäure,
- 4-[1-(1-Methoxy-2,2,2-trifluorethyl)-5,6,7,8-tetrahydro-5,5,8,8-tetramethyl-3-anthracenyl)-benzoesäure,
- 4-(5,5-Dimethyl-8-phenyl-5,6-dihydronaphthalin-2-ylethinyl)-benzoesäure.

4. Verwendung nach einem der Ansprüche 1 bis 3, **dadurch gekennzeichnet, dass** die pharmazeutische Zusammensetzung weitere Wirkstoffe enthält.

5. Verwendung nach einem der Ansprüche 1 bis 4, **dadurch gekennzeichnet, dass** die pharmazeutische Zusammensetzung einen kosmetisch oder pharmazeutisch akzeptablen und mit der ausgewählten Art der Verabreichung kompatiblen Träger enthält.

6. Verwendung nach einem der Ansprüche 1 bis 5, **dadurch gekennzeichnet, dass** die Zusammensetzung eine pharmazeutische Zusammensetzung ist und in Form von Tabletten, Gelkapseln, Dragees, Sirups, Suspensionen, Lösungen, Pulvern, Granulaten, Emulsionen, Mikrokügelchen oder Nanokügelchen oder Lipidvesikeln öder polymeren Vesikeln, die eine kontrollierte Freisetzung ermöglichen, in Form von Lösungen oder Suspensionen für die Perfusion oder Injektion vorliegen kann.

7. Verwendung nach Anspruch 6, **dadurch gekennzeichnet, dass** die Inhibitoren der Wirkung von Retinsäure in einer täglichen Dosis von etwa 0,01 mg/kg bis 100 mg/kg Körpergewicht in 1 bis 3 Einnahmen verabreicht werden.

8. Verwendung nach einem der Ansprüche 1 bis 5, **dadurch gekennzeichnet, dass** die pharmazeutische Zusammensetzung für eine Verabreichung auf topischem Wege geeignet ist und in Form von Lösungen oder Suspensionen, Salben, Pomaden, Cremes, Milchen, Gels, Pulvern, durchtränkten Tampons, Lotionen, Sprays, oder schäumenden oder reinigenden Produkten, in Form von Mikrokügelchen oder Nanokügelchen oder Lipidvesikeln oder polymeren Vesikeln oder in Form von polymeren Pflastern, Hydrogels oder eines Verbandes, die eine kontrollierte Freisetzung ermöglichen, oder in wasserfreier Form, in wässriger Form vorliegen kann, die außerdem Exzipientien enthalten können, die für die Erzeugung eines Filmes auf der Oberfläche der Haut geeignet sind.

9. Verwendung nach einem der Ansprüche 1 bis 5, **dadurch gekennzeichnet, dass** die Zusammensetzung eine Zusammensetzung ist, die für die Verabreichung im Bereich der Augen geeignet ist.

10. Verwendung nach einem der Ansprüche 8 oder 9, **dadurch gekennzeichnet, dass** die Menge des Inhibitors der Wirkung von Retinsäure im Bereich von 0,001 bis 5 Gew.-%, bezogen auf das Gesamtgewicht der Zusammensetzung, liegt.

11. Verwendung nach einem der Ansprüche 1 bis 10 für die Behandlung von chirurgischen oder traumatischen Wunden.

## Claims

1. Use of an inhibitor of the activity of retinoic acid for the preparation of a pharmaceutical composition intended to promote healing, in particular skin healing.

2. Use according to Claim 1, **characterized in that** the inhibitor of the activity of retinoic acid is an antagonist of retinoic acid.

3. Use according to Claim 2, **characterized in that** the antagonists of retinoic acid are selected from the following compounds:
- 4-[7-(1-adamantyl)-6-methoxyethoxymethoxy-2-naphthyl]benzoic acid,
- 4-{[5,6-dihydro-5,5-dimethyl-8-(4-methylphenyl)-2-naphthalenyl]ethynyl}benzoic acid,
- (E)-4-[2-(4,4-dimethyl-7-heptyloxy-1,1-dioxo-3,4-dihydro-2H-1-benzothiopyran-6-yl)propenyl]benzoic acid,
- 4-[4,5,7,8,9,10-hexahydro-7,7,10,10-tetramethyl-1-(3-pyridylmethyl)anthra[1,2-b]pyrrol-3-yl]benzoic acid,
- 4-[4,5,7,8,9,10-hexahydro-7,7,10,10-tetramethyl-1-(3-pyridylmethyl)thioanthra[1,2-b]pyrrol-3-yl]benzoic acid,
- 4-[4,5,7,8,9,10-hexahydro-7,7,10,10-tetramethyl-1-(3-pyridylmethyl)anthra[1,2-d]pyrazol-3-yl]benzoic acid,
- 4-[3-(diamantyl)-4-methoxybenzamidolbenaoic acid,
- 4-[3-(diamantyl)-4-methoxybenzoyloxy]benzoic acid,
- 4-(N-phenyl-5,6,7,8-tetrahydro-5,5,8,8-tetramethylnaphtho[2,3-d]imidazol-2-yl)benzoic acid,
- 4-(N-benzyl-5,6,7,8-tetrahydro-5,5,8,8-tetramethylnaphtho[2,3-d]imidazol-2-yl)benzoic acid,
- 4-(5H-7,8,9,10-tetrahydro-5,7,7,10,10-pentamethylbenzo[c]-naphtho[2,3-b][1,4]diazepin-3-yl)benzoic acid,
- 4-[1-(1-methoxy-2,2,2-trifluoroethyl)-5,6,7,8-tetrahydro-5,5,8,8-tetramethyl-3-anthracenyl]benzoic acid,
- 4-(5,5-dimethyl-8-phenyl-5,6-dihydronaphthalen-2-ylethynyl)benzoic acid.

4. Use according to one of Claims 1 to 3, **characterized in that** the pharmaceutical composition comprises other active compounds.

5. Use according to one of Claims 1 to 4, **characterized in that** the pharmaceutical composition comprises a cosmetically or pharmaceutically acceptable carrier compatible with the mode of administration selected.

6. Use according to one of Claims 1 to 5,
**characterized in that** the composition is a pharmaceutical composition, and may be provided in the form of tablets, gelatin capsules, sugar-coated tablets, syrups, suspensions, solutions, powders, granules, emulsions, lipid or polymeric microspheres or nanospheres or vesicles which allow a controlled release, solutions or suspensions for infusion or for injection.

7. Use according to Claim 6, **characterized in that** the inhibitors of the activity of retinoic acid are administered in a daily dose of about 0.01 mg/kg to 100 mg/kg as body weight, and this at the rate of 1 to 3 doses.

8. Use according to one of Claims 1 to 5, **characterized in that** the pharmaceutical composition is appropriate for administration by the topical route and may be provided in the form of solutions or suspensions, ointments, pommades, creams, milks, gels, powders, impregnated pads, lotions, sprays, or foaming or cleansing products, in the form of lipid or polymeric microspheres or nanospheres or vesicles or of polymeric patches, hydrogels or dressings allowing a controlled release, either in anhydrous form or in aqueous form, which may also comprise appropriate excipients for forming a film at the surface of the skin.

9. Use according to one of Claims 1 to 5, **characterized in that** the composition is a composition appropriate for administration by the ocular route.

10. Use according to either of Claims 8 and 9, **characterized in that** the quantity of inhibitor of the activity of retinoic acid is between 0.001% and 5% by weight relative to the total weight of the composition.

11. Use according to one of Claims 1 to 10, for the treatment of surgical or traumatic wounds.
